# EUROPEAN PATENT APPLICATION

(11) **EP 4 183 484 A1**
(43) Date of publication of application: **24.05.2023**
(21) Application number: 22208940.1
(22) Date of filing: 22.11.2022
(51) Int. Cl.: B01J 23/83, B01J 37/02, B01J 37/08, B01J 37/18, B01J 35/10, B01J 35/02, C07C 1/04

(54) **CATALYST FOR AND METHOD OF METHANATION OF CARBON MONOXIDE, CARBON DIOXIDE OR MIXTURES THEREOF, METHOD FOR PREPARING THE CATALYST AND DUAL-FUNCTION MATERIAL FOR THE ADSORPTION AND METHANATION**

(30) Priority: 22.11.2021 ES 202131088
(71) Applicant: Universidad del Pais Vasco - Euskal Herriko Unibertsitatea (UPV/EHU), 48940 Leioa (ES)
(72) Inventor: Onrubia Calvo, Jon Ander, Leioa (ES); Pereda Ayo, Beñat, Leioa (ES); González Marcos, José Antonio, Leioa (ES); González Velasco, Juan Ramón, Leioa (ES)
(74) Representative: Balder IP Law, S.L.

(57) **Abstract**

The present invention relates to a catalyst, such as Ni-La2O3/CeO2, for the methanation of carbon monoxide, carbon dioxide or mixtures thereof, comprising a support that comprises at least one metal oxide; at least one metal oxide of an element A, wherein the element A is a lanthanide element, an alkaline element, an alkaline earth element or mixtures thereof; and nanoparticles of an element B, wherein the element B is a noble metal, a transition metal or mixtures thereof, wherein said nanoparticles have a size between 1 nm and 8 nm and are distributed on the catalyst surface. The present invention also relates to the catalyst preparation method as defined above, the catalyst which can be obtained by said method and a method for the methanation of carbon monoxide, carbon dioxide or mixtures thereof, which comprises bringing the catalyst as defined above into contact with a flow stream of carbon monoxide, carbon dioxide or mixtures thereof and hydrogen at a temperature between 200 and 600 °C. Finally, the present invention relates to a dual function material for the adsorption and methanation of carbon monoxide, carbon dioxide or mixtures thereof from diluted or concentrated streams comprising the catalyst as defined above, and to the use of the catalyst as defined above in the adsorption and/or methanation of carbon monoxide, carbon dioxide or mixtures thereof. The perovskite-type catalyst precursor of the examples is LaNiO3 supported on either CeO2 or Al2O3

## Description

### TECHNICAL FIELD OF THE INVENTION

The present invention relates to the field of catalysis, and in particular to the field of catalysts for the methanation of carbon monoxide, carbon dioxide or mixtures thereof, the preparation method thereof and its application in "Power-to-Gas" technology.

### BACKGROUND OF THE INVENTION

The increase in global energy demand has caused a rapid growth in greenhouse gas emissions. In recent years, the greater awareness of citizens about the risks arising from global warming caused by greenhouse gas emissions has attracted widespread attention from social players and society itself. CO₂, one of the main components of greenhouse gases, contributes more than 60 % to global warming due to its huge amounts of emissions, which have exceeded 30 Gt per year during the last decade [J. Zhao et al., ACS Sustain. Chem. Eng., 2019, 17, 4831; J.M. Ketzer et al., Handbook of Climate Change Mitigation, Springer US, New York, NY, 2012, pp. 1405-1440]. Therefore, the need to mitigate CO₂ emissions into the environment has promoted processes that operate in a neutral carbon cycle. In this sense, the exploitation of CO₂ as a carbon source has received considerable attention in recent years. One of the most promising alternatives for recovering CO₂ is based on the concept known as "Power-to-Gas" (PtG). In this technology, CO₂ is hydrogenated into methane from H₂, obtained by water hydrolysis and using surplus electrical energy from renewable sources. Therefore, it helps to reduce the carbon footprint and mitigate renewable energy intermittency at the same time.

The reaction through which CO₂ is converted into methane is known as the Sabatier reaction [P. Sabatier, Comptes Rendus, 1902, 134, 514] and is characterised in that it is strongly exothermic and thermodynamically favoured at low temperature (ΔH⁰ = -164 KJ mol⁻¹). However, the reduction of the eight electrons of CO₂ to CH₄ with hydrogen has to overcome a kinetic barrier. To that end, the conversion of CO₂ into CH₄ usually speeds up at low temperatures, through the use of different catalytic formulations. Most of the catalysts are based on nickel or ruthenium as active phases. Nickel has a good cost/activity ratio, while ruthenium, despite its excellent activity and selectivity towards methane, has a higher cost, which limits its widespread application [A. Quindimil et al., Catal. Today, 2020, 356, 419; A. Bermejo-López et al., J. CO2 Util., 2019, 34, 576]. The simple synthesis and lower price of Ni-based catalysts, prepared by deposition methods (e.g., vapour infiltration and impregnation), have led to the fact that they have been the most widely researched in recent years [H. Arandiyan, Y et al., ACS Appl. Mater. Interfaces, 2018, 10, 16352]. However, conventional preparation methods often lead to a large and heterogeneous particle size distribution, which limits control over the interaction between the metal nanoparticles (NPs) and the support [L. Dai et al., Rare Metals, 2021, 40, 555]. This means that Ni-based catalysts are easily deactivated by sintering the metal at high temperatures. Furthermore, these catalysts usually have limited catalytic activity at low temperatures. Both drawbacks limit their widespread application on an industrial scale. Consequently, the design of Ni-based catalysts with high activity at low temperature and good stability is crucial to carry out the methanation reaction of CO₂ on a general industrial scale.

Researchers at Daihatsu and Toyota first proposed the exsolution of active phase NPs from a host oxide, such as a perovskite-type structure (ABO₃), with the aim of achieving a homogeneous distribution of the active centres and attaining greater control of their interactions with the support [Y. Nishihata, Nature, 2002, 418, 164]. In their study, palladium moved reversibly into/out of the LaFe_{0.57}Co_{0.38}Pd_{0.05}O₃ perovskite structure in the presence of redox atmospheres. Due to the small size of the extracted Pd NPs, this formulation maintained an activity as a three-way catalyst similar to that shown by the conventional formulation Pd/CeO₂-Al₂O₃, despite having a reduction in the noble metal proportion of 70-90 %. Gao et al. explored this concept to control the size and distribution of Ni particles in CO₂ methanation catalysts [Gao et al., J. Fuel Chem. Technol., 2009, 37, 573]. In their study, they proposed a LaNiO₃-type perovskite as a host oxide to carry out the exsolution of Ni NPs from inside the crystalline structure to the outside. However, there is still a wide margin for improvement in relation to stability and selectivity relative to that of the systems described in the state of the art. Furthermore, obtaining them by simple and cost-effective synthesis processes is also the subject of great interest.

More recent studies have been modifying the LaNiO₃ perovskite (LaNi_{0.08}Al_{0.92}O₃, La₁₋ₓCaₓNiO₃ and LaNi₁₋ₓMoₓO₃) with the aim of improving the properties of the catalyst [H. Arandiyan et al., ACS Appl. Mater. Interfaces, 2018, 10, 16352; J. Gao et al., Fuel Chem. Technol., 2009, 37, 573; T. Maneerung et al., J Hydrogen En., 2017, 42, 9840; E. Yang et al., 20th World Hydrogen Energy Conference, Proc. WHEC, 2014, 3, 1618; Lim HS et al., Chem. Eng. J., 2021, 412:127557], However, mass (unsupported) perovskites exhibited fairly low surface areas, which limits Ni dispersion and accessibility.

To address the aforementioned limitation, Wang et al., Li et al. and Zhang et al. distributed LaNiO₃-type perovskites on silica supports [X. Wang et al., Sustainable Chem. Eng., 2019, 7, 14647; S. Li et al., J. Hydrogen En., 2019, 44, 1597; T. Zhang et al., Energy Technol., 2020, 8, 1901164; T. Zhang et al. Int. J. Hydrogen Energy, 2020; 45 (7): 4417]. Alternatively, Li et al. supported this formulation on ZrO (La₁₋ₓCeₓNiO₃/ZrO₂) [G. Li et al., J. Energy Chem., 2020, 43, 155]. The catalysts obtained, after reducing these catalytic precursors, showed greater efficiency in CO₂ methanation and resistance to sintering than the catalysts obtained from mass perovskites.

Parallel to continuous CO₂ methanation on conventional systems, the use of Dual Function Materials (DFM) to carry out CO₂ methanation in diluted streams without the need for a prior sequestration and concentration step has been recently published [M. S. Duyar et al., Appl. Catal. B: Environ., 2015, 168-169, 370; P. Melo Bravo et al., Waste Disposal Sustain. Energy, 2019, 1, 53; S. Wang et al., J. CO2 Util., 2018, 27, 390; A. Bermejo-López et al., Appl. Catal. B: Environ., 2019, 256, 117845]. Usually, DFMs contain an alkali or alkaline earth metal (CaO or Na₂CO₃) for CO₂ adsorption and a noble metal (Ru) or transition metal (Ni) to promote the methanation reaction. The first study on applying dual function materials (DFMs) to carry out CO₂ methanation from diluted streams dates from 2015, since then, given that the process seems promising, the number of publications related to methanation in CO₂ adsorption and hydrogenation cycles using a DFM has grown significantly.

However, there is still a need to develop new active, selective and stable systems that boost the industrial application of "Power-to-Gas" technologies as an efficient way to store large amounts of intermittent energy produced from renewable sources for long periods of time.

### BRIEF DESCRIPTION OF THE INVENTION

The authors of the present invention have developed a catalyst for the methanation of carbon monoxide, carbon dioxide or a mixture thereof, through the controlled reduction and resulting decomposition of a supported ABO₃ perovskite, such that the final catalyst comprises a support that comprises at least one metal oxide; at least one oxide of the element that forms the A cation of the starting perovskite; and nanoparticles of the element that forms the B cation of the starting perovskite, with a size between 1 nm and 8 nm, in high interaction with the rest of the elements of the final catalyst and homogeneously distributed therein.

To that end, a first aspect of the present invention relates to a catalyst for the methanation of carbon monoxide, carbon dioxide or mixtures thereof, comprising:
- a support that comprises at least one metal oxide;
- at least one oxide of an element A, wherein the element A is a lanthanide element, an alkaline element, an alkaline earth element or mixtures thereof; and
- nanoparticles of an element B, wherein the element B is a noble metal, a transition metal or mixtures thereof, wherein said nanoparticles have a size between 1 nm and 8 nm and are distributed on the catalyst surface.

The catalyst of the present invention is prepared starting from a supported ABO₃ perovskite, which presents a high distribution of its particles on the support and which, after carrying out a controlled reduction of the same, leads to obtaining particles of the element that forms the B cation of the starting ABO₃ perovskite, of nanometric size and that are homogeneously distributed on the final catalyst. These features give rise to a significant reduction in the size of the catalyst active phase and an improvement in its interactions with the support with respect to conventional catalysts, which results in a synergistic effect different from that observed for catalysts based on the direct combination of, for example, Ni or Ru particles on Al₂O₃, La-Al₂O₃, CeO₂ or CeO₂-ZrO supports. Consequently, the catalyst of the present invention favours the selective catalytic hydrogenation of carbon monoxide, carbon dioxide or a mixture thereof to methane at significantly lower temperatures than conventional catalysts (e.g., Ni/CeO₂ or Ni/Al₂O₃) described in the state of the art (the latter reach similar conversion levels at temperatures between 15-100 °C higher).

Furthermore, the formulation of the catalyst of the present invention improves its durability and promotes a significant reduction in the content of the active phase, and, therefore, in its useful life and cost, which favours its widespread industrial application.

A second aspect of the present invention relates to the method for preparing the catalyst for the methanation of carbon monoxide, carbon dioxide or a mixture thereof, as defined above, comprising the steps of:
a) preparing an aqueous solution of metal precursors of an ABO₃ perovskite and citric acid, wherein the molar ratio of citrate to metal precursors is between 0.7 and 1.5, and wherein the pH of the aqueous solution is adjusted to a value between 1 and 9;
b) distributing the aqueous solution resulting from step (a) on a support by wet impregnation to obtain a supported perovskite precursor gel;
c) calcining the gel resulting from step (b) to obtain the supported perovskite; and
d) reducing the supported perovskite resulting from step (c) to give rise to the final catalyst.

The catalyst preparation method of the present invention enables supported perovskites, precursors of the final catalyst, highly dispersed on the support, to be obtained. The high distribution obtained promotes that, after the controlled reduction of this material, the exsolution of nanometric-sized particles of the element that forms the B cation of the starting ABO₃ perovskite is achieved, in high interaction with the rest of the elements of the catalyst. These features give rise to the aforementioned synergistic effect and selective catalytic activity in the hydrogenation of carbon monoxide, carbon dioxide or a mixture thereof at significantly lower temperatures than conventional catalysts.

To that end, a third aspect of the present invention relates to a catalyst for the methanation of carbon monoxide, carbon dioxide or a mixture thereof, which can be obtained by the method as defined above, comprising:
- a support that comprises at least one metal oxide;
- at least one oxide of an element that forms the A cation of a starting ABO₃ perovskite, wherein said element is a lanthanide element, an alkaline element, an alkaline earth element or mixtures thereof; and
- nanoparticles of an element that forms the B cation of a starting ABO₃ perovskite, wherein said element is a noble metal, a transition metal or mixtures thereof, wherein said nanoparticles have a size between 1 nm and 8 nm and are distributed on the catalyst surface.

Likewise, a fourth aspect of the present invention relates to a method for the methanation of carbon monoxide, carbon dioxide or mixtures thereof, which comprises bringing the catalyst as defined above into contact with a flow stream of carbon monoxide, carbon dioxide or mixtures thereof and hydrogen at a temperature between 200 and 600 °C.

The catalyst of the present invention also enables it to be easily adapted and applied as a dual function material for the adsorption and/or methanation of carbon monoxide, carbon dioxide or mixtures thereof, from both concentrated streams and diluted streams, and, therefore, without the need for a prior step of sequestration, purification and concentration. The catalyst of the present invention has shown high efficiency both in the adsorption of carbon monoxide, carbon dioxide or mixtures thereof and in the subsequent hydrogenation to methane.

To that end, a fifth aspect of the present invention relates to a dual function material for the adsorption and methanation of carbon monoxide, carbon dioxide or mixtures thereof from diluted or concentrated streams that comprises the catalyst as defined above.

A final aspect of the present invention relates to the use of the catalyst as defined above in the adsorption and/or methanation of carbon monoxide, carbon dioxide or mixtures thereof.

### BRIEF DESCRIPTION OF THE DRAWINGS

Figure 1 shows the spatial distribution of La, Ni and Ce, along with the Ni particle size distribution for the catalyst obtained after the reduction of the 10 % LaNiO₃/CeO₂ precursor.
Figure 2 shows a diagram of the methanation reaction with the Ni-La₂O₃/CeO₂ catalyst synthesised by reduction of a supported LaNiO₃/CeO₂ perovskite.
Figure 3 shows the distribution of carbon species at different temperatures for the catalysts obtained after the reduction of 10 % LaNiO₃/CeO₂ and 30 % LaNiO₃/La-Al₂O₃, LaNiO₃ and the 8.5 % Ni/CeO₂ conventional catalyst, for reference. H₂/CO₂ ratio=4, P=1 bar, space velocity (GHSV)=15,000 h⁻¹
Figure 4 shows a direct comparison of: a) CO₂ conversion and b) the selectivities towards methane and carbon monoxide of the catalyst obtained after the reduction of 10 % LaNiO₃/CeO₂ (10 % LNO/CeO₂) and the conventional catalyst (8.5 % Ni/CeO₂).
Figure 5 shows CO₂, CO, H₂O and CH₄ concentration profiles during a CO₂ adsorption and hydrogenation cycle at 400 °C for the catalyst obtained after the controlled reduction of the 10 % LaNiO₃/CeO₂ formulation.

### DETAILED DESCRIPTION OF THE INVENTION

Unless defined otherwise, all technical and scientific terms used in this document have the same meaning as the meaning commonly understood by a person skilled in the art to which this description pertains.

### Catalyst of the present invention

As defined above, in a first aspect, the present invention relates to a catalyst for the methanation of carbon monoxide, carbon dioxide or a mixture thereof, comprising:
- a support that comprises at least one metal oxide;
- at least one oxide of an element A, wherein the element A is a lanthanide element, an alkaline element, an alkaline earth element or mixtures thereof; and
- nanoparticles of an element B, wherein the element B is a noble metal, a transition metal or mixtures thereof, wherein said nanoparticles have a size between 1 nm and 8 nm and are distributed on the catalyst surface.

In the context of the present invention, the term "methanation" refers to the hydrogenation of carbon dioxide to methane, a reaction known as the Sabatier reaction (1), starting from hydrogen produced preferably with surplus electrical energy from renewable sources ("Power-to-Gas" (PtG)).

CO₂ + H₂ ↔ CH₄ + H₂O (1)

In the context of the present invention, the term "methanation" also encompasses the hydrogenation of carbon monoxide. The methanation reaction of carbon dioxide is, furthermore, a product of the combination of the reaction known as "Reverse Water Gas Shift" (2) and the methanation reaction of carbon monoxide (3).

H₂ + CO₂ ↔ CO + H₂O (2)

3H₂ + CO ↔ CH₄ + H₂O (3)

Therefore, the catalyst of the present invention is applicable for the methanation of both carbon monoxide and carbon dioxide and mixtures thereof.

The main sources of carbon dioxide, carbon monoxide and mixtures thereof are the processes of coal, oil and gas combustion from power plants, automobiles and industrial facilities.

As defined above, the catalyst of the present invention comprises a support that comprises at least one metal oxide.

In the context of the present invention, the term "support" refers to a material, usually a solid with a high surface area, on which the active phase of a catalyst is fixed. The support used in the catalyst of the present invention comprises at least one metal oxide, on which the starting ABO₃ perovskite is supported, which is subsequently reduced to obtain the final catalyst. After the reduction of said perovskite, an oxide of the element that forms the A cation of the starting perovskite and nanoparticles of the element that forms the B cation of the starting perovskite are obtained. Both species partially or totally cover the surface of the support and are in high interaction with it.

Suitable supports for the catalyst of the present invention are commercial supports or supports prepared by methods known in the technical field of the invention such as precursor calcination, precipitation, etc. A non-limiting example of the above is a ceria support (CeO₂) obtained by the direct calcination of the Ce(NO₃)₃ . 6H₂O precursor.

Other non-limiting examples of suitable supports for the catalyst of the present invention are supports that comprise group III metal oxides, group IV metal oxides, lanthanides, rare earth elements and mixed oxides thereof such as, for example, SiO₂-Al₂O₃, zeolites or CeO₂-ZrO₂.

Alumina-containing supports can be, for example, γ-alumina, zeolites or hydrated aluminosilicates. Zeolites can be natural zeolites or synthetic zeolites (Y, X, ZSM-5, etc.), with hierarchical porosity or with different Si/AI ratios. Furthermore, supports that comprise group III metal oxides, group IV metal oxides, lanthanides or rare earth elements can be modified or passivated with one or more elements, such as La, Ti, Zr, Ce, etc., as well as mixtures of said elements.

Next, the specific surface area (SBET), the pore volume (Vp) and the pore diameter (dp) of 3 mesoporous supports suitable for the catalyst of the present invention are shown as a non-limiting example. These supports are: commercial γ-alumina, ceria obtained by direct calcination of the cerium nitrate precursor, and La-alumina obtained after incorporating lanthanum by impregnation on commercial γ-alumina.

| **Sample** | **S_{BET}, m² g⁻¹** | **Vₚ, cm³ g⁻¹** | ***dp, nm*** |
|---|---|---|---|
| CeO₂ | 78.3 | 0.16 | 6.85 |
| Al₂O₃ | 193 | 0.54 | 8.56 |
| La-Al₂O₃ | 179 | 0.48 | 8.26 |

In a particular embodiment, the at least one metal oxide of the support is selected from γ-Al₂O₃, γ-Al₂O₃ passivated with La, CeO₂ and CeO₂-ZrO₂.

In a particular embodiment, the support represents between 90 % and 50 % by weight with respect to the total weight of the final catalyst.

As previously mentioned, the catalyst of the present invention further comprises at least one oxide of an element A, wherein the element A is a lanthanide element, an alkaline element, an alkaline earth element or mixtures thereof. This oxide is the result of the controlled reduction of a starting ABO₃ perovskite, wherein A is the element that forms the A cation of the starting perovskite.

In the context of the present invention, the term "element" refers to a chemical element in the periodic table.

Non-limiting examples of elements in the periodic table that can form the A cation of the starting perovskite and, therefore, the element A of the oxide of the final catalyst are La, Ba, Ca, Sr, Na, K and Ce. Therefore, non-limiting examples of metal oxides of the element A suitable for the catalyst of the present invention are La₂O₃, BaO, CaO, SrO, Na₂O, K₂O, CeO and mixtures thereof.

The oxide of the element A is homogeneously distributed on the surface of the support. In a particular embodiment, the oxide of the element A represents between 2,8 % and 56 % by weight with respect to the total weight of the catalyst.

The catalyst of the present invention further comprises nanoparticles of an element B, wherein the element B is preferably in the zero-oxidation state and is a noble metal, a transition metal or mixtures thereof. The element B corresponds to the element that forms the B cation of the starting ABO₃ perovskite.

Non-limiting examples of elements in the periodic table that can form the B cation of the starting perovskite and, therefore, the element B of the nanoparticles of the final catalyst are Cu, Co, Mn, Fe, Ni, Ru, Rh, Pt, Pd, Au, Ag, Al, Mo and mixtures thereof. Therefore, non-limiting examples of nanoparticles of the element B are Cu, Co, Mn, Fe, Ni, Ru, Rh, Pt, Pd, Au, Ag, Al, Mo nanoparticles and mixtures thereof, wherein said chemical elements are found, essentially, in the zero-oxidation state.

Nanoparticles of the element B have a size between 1 nm and 8 nm, preferably less than 5 nm.

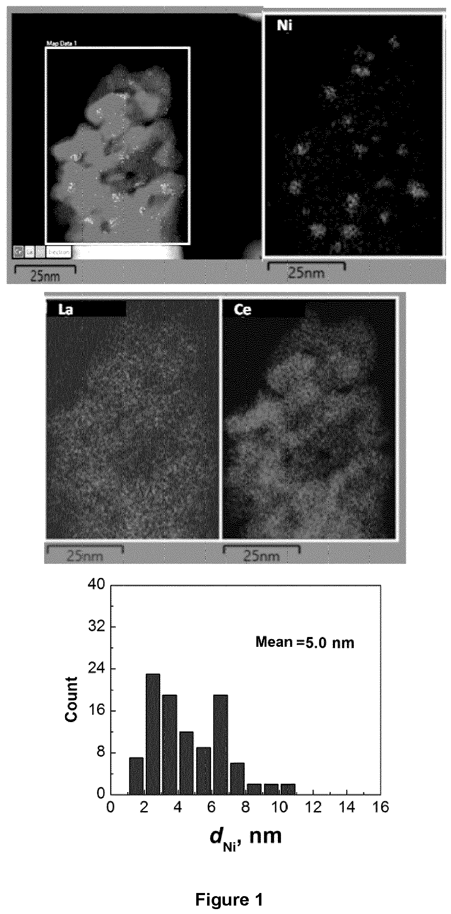

In the context of the present invention, the term "size" in relation to the nanoparticles of the element B refers to the mean diameter of the particle that is obtained by the usual techniques in the technical field of the present invention, and through which similar results are obtained. Non-limiting examples of these techniques are the Scanning Transmission Electron Microscopy technique (e.g., Figure 1), from which the size of more than 100 particles in the obtained images can be measured and on which the mean particle size is calculated; the hydrogen desorption technique at a programmed temperature, which enables desorbed hydrogen to be determined, which enables the surface nanoparticles, their dispersion and their mean size to be determined; or the hydrogen chemisorption technique using hydrogen adsorption isotherms.

Nanoparticles of the element B are obtained after the controlled reduction of a starting perovskite (ABO₃). The nanoparticles of the element B are in high interaction with the rest of the elements of the catalyst. The authors of the present invention have observed that the exsolution of nanoparticles of the element that forms the B cation of the starting perovskite results in a synergistic effect between the components of the catalyst, which is different from that observed for catalysts based on the direct combination of, for example, Ni or Ru as an active phase and Al₂O₃, La-Al₂O₃, CeO₂ or CeO₂-ZrO as a support. Consequently, the catalysts provided by the present invention favour the selective catalytic hydrogenation of carbon dioxide, carbon monoxide and mixtures thereof to methane at significantly lower temperatures than conventional catalysts. Without being bound by any theory in particular, this synergistic effect is believed to be due to an optimal interaction of the active phase (nanoparticles extracted from the B cation of the starting perovskite) with the rest of the components of the catalyst (oxide extracted from the A cation of the starting perovskite and the support), which leads to a significant improvement in the stability and durability of the catalyst of the present invention with respect to conventional catalysts. Furthermore, the catalyst of the present invention enables the content of the active phase (element B that forms the B cation of the starting perovskite, such as, for example, Ni and/or Ru) to be significantly reduced with respect to conventional catalysts (direct combination of active phase and support) and, therefore, the cost is reduced, which improves its widespread industrial application.

In a preferred embodiment, the catalyst of the present invention comprises between 1.2 and 24 % by weight, with respect to the total weight of the catalyst, of nanoparticles of the element B, evenly distributed on the catalyst surface.

### Catalyst preparation method

In a second aspect, the present invention relates to the catalyst preparation method as defined above, comprising the steps of:
a) preparing an aqueous solution of metal precursors of an ABO₃ perovskite and citric acid, wherein the molar ratio of citrate to metal precursors is between 0.7 and 1.5, and wherein the pH of the aqueous solution is adjusted to a value between 1 and 9;
b) distributing the aqueous solution resulting from step (a) on a support by wet impregnation to obtain a supported perovskite precursor gel;
c) calcining the gel resulting from step (b) to obtain the supported perovskite; and
d) reducing the supported perovskite resulting from step (c) to give rise to the final catalyst.

The catalyst preparation method of the present invention consists of the combination of the citric acid sol-gel method and the wet impregnation method. By combining both methods (in steps (a)-(c) of the method of the present invention), the ABO₃ perovskite is formed on the selected support. Once the perovskite is formed, a controlled reduction of the same is carried out (step (d) of the method of the present invention) to obtain the final catalyst. As a result of this reduction process, the ABO₃ perovskite structure obtained in step (c) is destroyed by the exsolution extraction of the element that forms the B cation thereof. Furthermore, by means of said controlled exsolution, the size of the active phase particles is controlled. At the end of the method of the present invention, a catalyst is obtained that comprises at least one oxide of the element that forms the A cation of the starting perovskite homogeneously distributed on the support; and nanoparticles of the element that forms the B cation of the starting perovskite, smaller in size than in conventional catalysts and with high dispersion on the catalyst surface. Furthermore, both elements A and B are in high interaction with each other and with the support, in the final catalyst.

In particular, step (a) of the catalyst preparation method of the present invention comprises preparing an aqueous solution of metal precursors of an ABO₃ perovskite and citric acid, wherein the molar ratio of citrate to metal precursors is between 0.7 and 1.5, and wherein the pH of the aqueous solution is adjusted to a value between 1 and 9.

In the context of the present invention, the citric acid method (also called citric acid sol-gel method or citrate sol-gel method; Baythoun, M.S.G., Sale, F.R. Production of strontium-substituted lanthanum manganite perovskite powder by the amorphous citrate process. J Mater Sci 17, 2757-2769 (1982)) relates to the method that is commonly used for the synthesis of binary, ternary and quaternary metal oxides in crystalline and amorphous forms. This method consists of using citric acid or other similar carboxylic acids as a complexing agent that is mixed with a precursor of the metal cations of the metal oxide (in the case of ABO₃ perovskite, they are precursors of the A and B metal cations). In the present invention, said mixture is subjected to controlled calcination, in 5 % O₂/He flow, at a high temperature (600-1200 °C), to purge the gases released during calcination, to avoid the formation of hot spots during calcination that cause the agglomeration of the perovskite, and to carry out the spontaneous combustion process progressively, reducing the O₂ content with respect to the air content.

In the context of the present invention, the term "perovskite" refers to any material that has the same type of crystalline structure as calcium titanate (CaTiO₃), known as a perovskite structure, or ^{XII}A^{2+VI}B⁴⁺X²⁻₃ wherein X is oxygen (perovskite oxide), which is arranged in the centres of the faces. The general chemical formula of the perovskite compounds used in the present invention is ABO₃, wherein A and B are cations of different sizes and O is an oxygen anion bound to both. The A atoms are larger than the B atoms. Normally the A and B cations will have a +3/+4 oxidation state in the perovskite, even if it is not the most common oxidation state of the element in question.

Non-limiting examples of metal precursors of the ABO₃ perovskite suitable for step (a) of the method as defined above are metal nitrates, metal chlorides, metal acetates and mixtures thereof of the elements A and B of the ABO₃ perovskite to be obtained. In a preferred embodiment, the perovskite precursor is a metal nitrate or a mixture of several.

The A cation comprises a lanthanide element, an alkaline element, an alkaline earth element or mixtures thereof. Non-limiting examples of suitable A cations for the present invention are La³⁺, Ba²⁺, Ca²⁺, Sr²⁺, Na⁺, K⁺ ad Ce^{3/4+} cations.

The B cation comprises a noble metal, a transition metal or mixtures thereof. Non-limiting examples of suitable B cations for the present invention are noble metal and transition metal cations, such as Cu²⁺, Co³⁺, Mn^{3/4+}, Fe³⁺, Ni³⁺, Ru⁴⁺, Rh³⁺, Pt⁴⁺, Pd²⁺, Au³⁺, Ag⁺, Al³⁺ and Mo⁶⁺ cations.

Therefore, non-limiting examples of metal precursors suitable for step (a) of the method are La(NO₃)₂ and Ni(NO₃)₂.

The authors of the present invention have observed that the control of the molar ratio of citric acid with respect to the metal precursors (metal cation) between 0.7 and 1.5 in step (a) favours a more homogeneous distribution of the different elements of perovskite, improves its porosity and reduces the crystal size thereof, which improves the physical and structural properties of perovskite. Lastly, this contributes to reducing the size of the exsolved nanoparticles of perovskite, which are obtained in step (d) of the method of the present invention.

In a preferred embodiment, the molar ratio of the citrate anion to the metal cations in step (a) is 1.1.

The authors of the present invention have also observed that the adjustment of the pH of the aqueous solution in step (a) between 1 and 9 favours a more homogeneous distribution of the different components of perovskite and a greater stability of perovskite, avoiding the formation of impurities.

In another preferred embodiment, the pH of step (a) is adjusted to a value between 5 and 9. The pH of step (a) can be adjusted by adding an ammonia solution (strong base), dropwise.

An exemplary embodiment of step (a) of the method of the invention is to dissolve stoichiometric amounts of different A metal cation precursors (for example, La cations with different degrees of substitution by Ce, Ca, Na, Ba and/or K cations) and B metal cation precursors (Ni cations with different degrees of substitution by Ru, Co, Fe and/or Mn cations), of the ABO₃ perovskite to be obtained, in the form of metal nitrates, in distilled water with vigorous stirring. Subsequently, citric acid is added as a complexing agent with a molar ratio of citrate to total metal nitrates of around 1 and the pH value is adjusted with an ammonia solution to values between 1 and 9.

Step (b) of the catalyst preparation method of the present invention comprises distributing the aqueous solution resulting from step (a) on a high surface support by wet impregnation to obtain a perovskite precursor gel evenly distributed on the support.

In the context of the present invention, the wet impregnation method relates to the method that is commonly used to add the active phase to the surface of a support in the preparation of heterogeneous catalysts [Geus, J.W. and van Dillen, A.J. (1999). Preparation of Supported Catalysts by Deposition-Precipitation. In Preparation of Solid Catalysts (eds G. Ertl, H. Knözinger and J. Weitkamp)]. The precursors are incorporated into the support by precipitation or crystallisation on the same, from a metal salt solution, usually aqueous, evaporating the solvent to dryness.

In a particular embodiment, the supported perovskite precursor gel resulting from step (b) is dried at a temperature between 80 °C and 150 °C, preferably between 80 °C and 140 °C, even more preferably at 120 °C. The drying of step (b) must be carried out during a period of time that ensures the loss of surface and combined water, i.e., preferably for at least 6 hours, more preferably between 6 and 12 hours.

An exemplary embodiment of step (b) of the method of the invention comprises that the aqueous solution resulting from step (a) is impregnated on a support (for example, a ceria support) and slowly evaporated until obtaining a precursor gel homogeneously distributed on the support. The obtained gel is dried at 120 °C for 12 h. Nominal loads of perovskite precursor of between 10 % and 50 % by weight with respect to the weight of the support are prepared.

Subsequently, in step (c) of the method of the present invention, the gel resulting from step (b) is calcined to obtain the supported perovskite.

As defined above, the general chemical formula of the perovskite obtained in the method of the present invention is ABO₃. In a particular embodiment, the perovskite obtained in the method of the present invention is represented by the formula A₁₋ₓA'ₓB_{1-y}B'_{y}O₃, wherein:
- A is La³⁺,
- A' is a cation of a lanthanide element, an alkaline element, an alkaline earth element or mixtures thereof, preferably selected from Ce³⁺, Ca²⁺, Ba²⁺, Na⁺ and K+,
- B is Ni³⁺,
- B' is a cation of a noble metal, a transition metal or mixtures thereof, preferably it is selected from Ru⁴⁺, Fe³⁺, Co³⁺ and Mn^{3/4+},
- x is between 0 and 0.5, and
- y is between 0 and 0.5.

In a particular embodiment, the supported perovskite represents between 10 % and 50 % by weight with respect to the total weight of the material obtained in step (c).

In a preferred embodiment, the calcination of step (c) is carried out at a temperature between 600 °C and 1200 °C, preferably at 600 °C. The calcination of step (c) should preferably be carried out for a period of time of at least 4 hours.

In another preferred embodiment, the calcination of step (c) is carried out under flow conditions, for example, with an O₂/He flow.

In an exemplary embodiment of step (c), the gel resulting from step (b) is calcined at a temperature between 600 °C and 700 °C for 4 h under a 5 % O₂/He flow to obtain the supported perovskite.

The supported perovskite obtained in step (c) of the method of the present invention has particle sizes between 20 nm and 50 nm.

The catalyst preparation method of the present invention further comprises a step (d) of reducing the supported perovskite resulting from step (c) to give rise to the final catalyst.

As a result of the reduction process of step (d) of the method of the present invention, the ABO₃ perovskite structure obtained in step (c) is destroyed by the exsolution extraction of the element that forms the B cation thereof. By means of said exsolution, the size of the active phase particles (element B that corresponds to the element that forms the B cation of the starting perovskite) is controlled.

At the end of the method of the present invention, a catalyst is obtained that comprises at least one oxide of the element that forms the A cation of the starting perovskite, which is homogeneously distributed on the support; and nanoparticles, essentially, in the zero oxidation state of the element that forms the B cation of the starting perovskite, smaller in size than in conventional catalysts (between 1 and 8 nm), and with high dispersion on the catalyst surface.

In an exemplary embodiment of the present invention, during step (d) of reducing the LaNiO₃/CeO₂ supported perovskite wherein the LaNiO₃ perovskite represents 30 % by weight of the total weight of the supported perovskite, the perovskite-type oxide is progressively reduced. Consequently, Ni is extracted in a controlled manner from the "host" perovskite, which favours the formation of Ni nanoparticles of a much smaller size and a more homogeneous distribution than in conventional catalysts prepared by direct deposition of Ni on a CeO₂ support (ceria). Furthermore, Ni goes progressively from an oxidation state of +3 (in the perovskite) to 0 (as nanoparticles), as shown in the following reactions:

4LaNiO₃ + 2H₂ → La₄Ni₃O₁₀ + Ni⁰ + 2H₂O (4)

La₄Ni₃O₁₀ + 3H₂ → La₂NiO₄ + 2Ni⁰ + La₂O₃ + 2H₂O (5)

La₂NiO₄ + H₂ → Ni⁰ + La₂O₃ + H₂O (6)

When step (d) of reduction is finished, the starting perovskite is completely destroyed, obtaining the nanoparticles of Ni⁰ and La₂O₃, both species extracted from the same. Since the reduction is carried out under controlled conditions, Ni is progressively exsolved from the perovskite, which enables its size to be controlled and a homogeneous distribution of the same to be favoured. Furthermore, since it forms part of the starting perovskite structure, together with La, it enables both species (Ni⁰ and La₂O₃) to be in high interaction with each other and with the ceria support in the final catalyst. This leads to a synergistic effect different from that observed for catalysts based on the direct combination of, for example, Ni particles on CeO₂ supports.

In a particular embodiment, the reduction of step (d) of the method of the present invention is carried out with a reducing flow stream, more particularly with a H₂/He, H₂/Ar or H₂/N₂ flow stream, wherein the H₂:inert gas ratio can be comprised between 10:1 and 100:1.

The selection of the temperature at which step (d) of reduction is carried out depends on the type of support used, so that it is high enough to lead to the complete exsolution of the elements of perovskite, but without being excessively high to prevent its sintering.

Non-limiting examples of temperatures used in step (d) of the method of the present invention are 550 °C for ceria-supported catalysts and 800 °C for alumina and La-alumina-supported catalysts.

In a particular embodiment, the reduction of step (d) of the method of the present invention is carried out at a temperature between 500 °C and 900 °C.

In an exemplary embodiment of step (d), the supported perovskite resulting from step (c) is subjected to a controlled reduction protocol with a flow stream of 20 % H₂/He at a temperature between 500 °C and 650 °C for 2 h in order to obtain the final catalyst.

The final catalyst resulting from step (d) is a solid with a high surface area (e.g., between 25 and 60 m²/g for ceria-supported catalysts, > 100 m²/g for alumina-supported catalysts, and even higher for zeolite-supported catalysts), with a high distribution of its components (oxide of the element A and nanoparticles of the element B) on the support.

The final catalyst resulting from step (d) comprises between 2.5 % and 27 % of the total weight of the catalyst of nanoparticles of the element B of perovskite, evenly distributed on the catalyst surface.

The authors of the present invention have observed a synergistic effect between the components of the final catalyst, which favours selective catalytic activity in the hydrogenation of carbon monoxide, carbon dioxide or a mixture thereof at temperatures significantly lower than conventional catalysts, as well as improved stability and, therefore, improved durability. Without being bound by any theory in particular, this synergistic effect and the close proximity of the active centres of the catalyst are believed to promote CO₂ adsorption, H₂ dissociation and the reduction of CO₂ adsorbed to methane. Furthermore, the high interaction of the different components of the catalyst improves the stability of the B nanoparticles with respect to conventional catalysts, significantly improving the durability of the catalyst, which is highly desirable for its application at an industrial level.

To that end, a third aspect of the present invention relates to a catalyst for the methanation of carbon monoxide, carbon dioxide or a mixture thereof which can be obtained by the method as defined above.

The catalyst for the methanation of carbon monoxide, carbon dioxide or a mixture thereof which can be obtained by the method as defined above comprises:
- a support that comprises at least one metal oxide;
- at least one oxide of an element that forms the A cation of a starting ABO₃ perovskite, wherein said element is a lanthanide element, an alkaline element, an alkaline earth element or mixtures thereof; and
- nanoparticles of an element that forms the B cation of a starting ABO₃ perovskite, wherein said element is a noble metal, a transition metal or mixtures thereof, wherein said nanoparticles have a size between 1 nm and 8 nm and are distributed on the catalyst surface.

### Applications of the catalyst element of the present invention

The catalyst of the present invention has great potential in the methanation of carbon monoxide, carbon dioxide or mixtures thereof.

To that end, a fourth aspect of the present invention relates to a method for the methanation of carbon monoxide, carbon dioxide or mixtures thereof from a wide variety of sources that comprises bringing the catalyst as defined above into contact with a flow stream of carbon monoxide, carbon dioxide or mixtures thereof and hydrogen at a temperature between 200 and 600 °C.

The methanation method of the present invention can be carried out both continuously and discontinuously, for example, in a fixed bed reactor or several fixed bed reactors connected in parallel in which the catalyst of the present invention is placed.

In a particular embodiment, the methanation method of the present invention is carried out with a molar ratio of H₂ to CO₂ (or CO or a mixture thereof) of ≥ 4:1.

In another particular embodiment, the methanation method of the present invention is carried out at a temperature between 200 °C and 600 °C, preferably between 300 °C and 350 °C.

In another particular embodiment, the methanation method of the present invention is carried out at a pressure between 1 atm and 50 atm.

In another particular embodiment, the methanation method of the present invention is carried out at a space velocity (GHSV) between 10,000 h⁻¹ and 100,000 h⁻¹.

CH₄ and CO concentrations are measured at the reactor outlet with a gas chromatograph. The authors of the present invention have observed that the catalyst of the present invention reaches a conversion of between 1 % and 80 % of CO₂. Furthermore, they have observed that the selectivity towards methane was maintained around 100 % throughout the temperature range and the loss of activity was less than 5 % after 72 hours of reaction.

The catalyst of the present invention can be used in the conversion of CO₂ (or CO or mixtures thereof) to methane both under continuous operating conditions (concentrated streams) or discontinuous operating conditions (diluted streams) via capture cycles (CO₂ adsorption) and methanation (hydrogenation), in other words, operating as a dual function material (DFM).

To that end, another aspect of the present invention relates to a dual function material for the adsorption and methanation of carbon monoxide, carbon dioxide or mixtures thereof, from diluted or concentrated streams, comprising the catalyst as defined above. These materials make it possible to carry out the methanation of CO₂, from streams of a different nature, without the need for a prior step of sequestration, purification and concentration.

Dual function materials operate in such a way that the operating conditions are changed at regular intervals to switch between the capture mode (adsorption) and the methanation mode (hydrogenation). During the adsorption step, a gas stream containing CO₂ (or CO or mixtures thereof) is applied in the reactor and the CO₂ (or CO or a mixture thereof) is stored as carbonates at the basic sites provided by the catalyst (usually, alkali or alkaline earth metals). When the catalyst is saturated with CO₂ (or CO or a mixture thereof), a hydrogen stream is applied to promote the decomposition of the carbonates and their subsequent hydrogenation to CH₄, the active phase of the catalyst.

To that end, a final aspect of the present invention relates to the use of the catalyst as defined above in the adsorption and/or methanation of carbon monoxide, carbon dioxide or mixtures thereof.

### EXAMPLES

### Example 1 - Preparation of the Ni-La₂O₃/CeO₂ catalyst from a starting supported perovskite with a nominal load of 10 %.

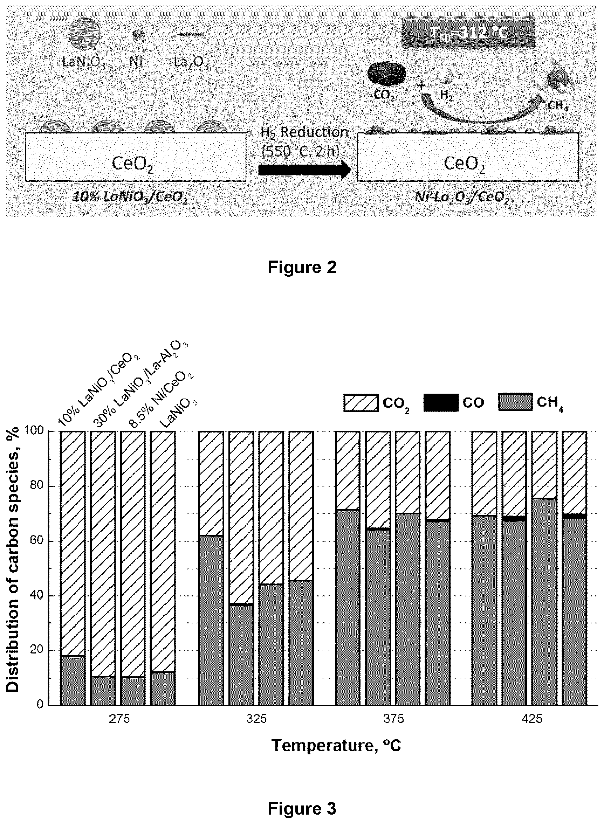

First, the ceria support was obtained by direct calcination of the Ce(NO₃)₃ · 6H₂O precursor (Sigma Aldrich, 99,9 %). Once the CeO₂ support was obtained, stoichiometric amounts of La(NO₃)₂ · 6H₂O (Merck, 99,0 %) and Ni(NO₃)₂ · 6H₂O (VWR, 99,9 %) were dissolved in distilled water with vigorous stirring, 1.76 and 1.18 g, respectively. Subsequently, citric acid (C₆H₈O₇ · H₂O, Sigma Aldrich, 99 %) was added as a complexing agent with a molar ratio of citrate to metal nitrates of 1.1 (1.72 g) and the pH value was adjusted with an ammonia solution (25 % as NH₃, Panreac) to a value of 7. The resulting solution was added to the ceria support (9 g) inside a rotary evaporator (under vacuum and at 35 °C), wherein the controlled evaporation of the solution was carried out until a precursor gel homogeneously distributed on the ceria support was obtained. The obtained gel was dried at 120 °C for 12 h. The formation of perovskite on CeO₂ was achieved by calcining the sample at 600 °C for 4 h under a flow of 5 % O₂/He in a horizontal tube furnace. A formulation with a nominal perovskite content of 10 % by weight on CeO₂ was prepared. To finally obtain the catalyst, the catalytic precursor was subjected to a controlled reduction protocol in 20 % H₂/He at 550 °C for 2 h. As a result of this process, the LaNiO₃ perovskite was progressively reduced, leading to the exsolution of nanometre-sized Ni nanoparticles (1-5 nm, **Figure 1**) in close contact with the support and La₂O₃. In this manner, after this step, a Ni-La₂O₃/CeO₂-type catalyst was obtained (**Figure 2**).

The distribution of the different phases present in the sample is analysed by scanning transmission electron microscopy (STEM) together with energy-dispersive X-ray spectroscopy (EDS). Figure 1 includes the STEM images and the corresponding EDS elemental mapping of Ni, La and Ce for a 10 % LaNiO₃/CeO₂ sample, after subjecting them to controlled reduction and CO₂ methanation reaction. Furthermore, histograms obtained for the Ni particle size distribution are included. As a general trend, the elements Ce and La coexist with a homogeneous distribution on all the areas analysed. Small Ni nanoparticles, obtained after reduction and destruction of LaNiO₃, are evenly distributed on its surface and have a homogeneous size. In accordance with the Ni size estimated by H₂-TPD and XRD experiments, the average size of the nanoparticles is the size of Ni (2.5 nm). These results suggest that the smaller particle size (20-30 nm), observed for the 10 % LaNiO₃/CeO₂ precursor, favours the formation of Ni-La₂O₃/CeO₂ catalysts with highly dispersed Ni nanoparticles in close contact with the La₂O₃ and CeO₂ phases, after controlled reduction of the precursor. The formation of these intermediate phases (Ni-CeO₂ and Ni-La₂O₃) contributes to preventing thermal agglomeration of the Ni nanoparticles during calcination and the methanation reaction.

### Example 2 - Preparation of the Ni-La₂O₃/La-Al₂O₃ catalyst from a starting supported perovskite with a nominal load of 30 %.

First, the 5 % La-Al₂O₃ support was obtained by impregnation of La(NO₃)₂ · 6H₂O (Merck, 99,0 %) on γ-Al₂O₃. Next, stoichiometric amounts of La(NO₃)₂ · 6H₂O (Merck, 99,0 %) and Ni(NO₃)₂ · 6H₂O (VWR, 99,9 %) were dissolved in distilled water with vigorous stirring, 5.29 and 3.55 g, respectively. Subsequently, citric acid (C₆H₈O₇ · H₂O, Sigma Aldrich, 99 %) was added as a complexing agent with a molar ratio of citrate to metal nitrates of 1.1 (5.16 g) and the pH value was adjusted with an ammonia solution (25 % as NH₃, Panreac) to a value of 7. The resulting solution was impregnated on the 5 % La-Al₂O₃ support (7 g) inside a rotary evaporator (under vacuum and at 35 °C), wherein the controlled evaporation of the solution was carried out until a precursor gel homogeneously distributed on the support was obtained. The obtained gel was dried at 120 °C for 12 h and the resulting sample was calcined at 600 °C for 4 h under a flow of 5 % O₂/He for the formation of supported perovskite on 5 % La-Al₂O₃. The final catalyst was obtained by subjecting the catalytic precursor to a controlled reduction protocol in 20 % H₂/He at 800 °C for 2 h. As a result of this process, the LaNiO₃ perovskite was progressively reduced, leading to the exsolution of nanometre-sized Ni nanoparticles (3-8 nm) in close contact with the support and La₂O₃. In this manner, after this step, a Ni-La₂O₃/ La-Al₂O₃-type catalyst is obtained.

### Example 3 - Methanation reaction

CO₂ and CO methanation reactions were performed in a downstream fixed bed reactor (Din = 9 mm) at atmospheric pressure (1 atm). To that end, 0.5 g (dp = 0.3-0.5 mm) of the catalyst were mixed with quartz particles (0.5-0.8 mm) to a total volume of 1 cm³. The CO₂ and CO methanation reaction was carried out in a mixture of H₂/CO₂/He with a molar ratio of 4:1:1.25 (250 cm³ min⁻¹). These conditions result in a space velocity (GHSV) of 15,000 h⁻¹ and a space time (W/FA0) of 4.67 g_{cat} · h · mol⁻¹. During the catalytic test, the temperature was increased from 200 to 500 °C, in steps of 25 °C, with a heating rate of 5 °C min⁻¹ between each step. He, H₂, CO₂, CO and CH₄ concentrations at the reactor outlet were measured with a gas chromatograph once steady state was reached at each temperature. The catalysts synthesised in examples 1 and 2 reached a 50 % conversion of CO₂ at 312 °C and 345 °C (**Figure 3**), respectively. The selectivity towards methane was maintained around 100 % throughout the temperature range and the loss of activity was less than 5 % after 72 h of reaction at 300 °C in both cases.

**Figure 3** also includes a comparison with respect to a conventional catalyst containing ceria-supported Ni supported on ceria (8,5 % Ni/CeO₂), similar to those used industrially, and with respect to the LaNiO₃ perovskite without support.

The improvement in the conversion of carbon dioxide to methane in the catalysts of the present invention is especially evident at lower temperatures.

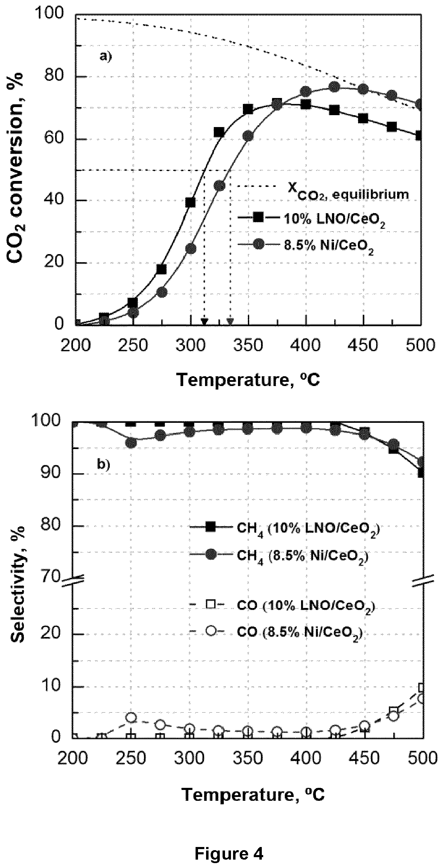

**Figure 4** also includes a direct comparison of the catalyst of example 1 (Ni-La₂O₃/CeO₂ referred to in said figure as 10 % LNO/CeO₂) and the 8,5 % Ni/CeO₂ conventional catalyst, similar to those used industrially. The improvement in the conversion of carbon dioxide to methane is especially evident at lower temperatures, which further enhances the benefit of the synergistic effect achieved as a result of the preparation method developed in the invention. In fact, T₅₀ (temperature at which 50 % conversion of CO₂ is reached) is approximately 20 °C (312 °C vs. 333 °C) lower for the developed alternative. In addition, the selectivity to methane is higher throughout temperature range under study.

### Example 4 - CO₂ adsorption and hydrogenation

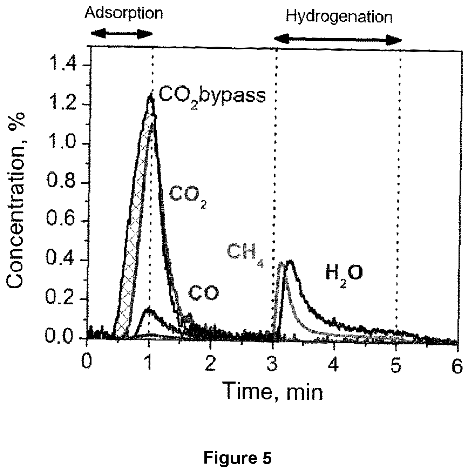

The catalysts of the present invention also demonstrated high efficiency as dual function materials (DFM), both in CO₂ adsorption and in its hydrogenation to methane (**Figure 5**).

CO₂ and CO methanation reactions were performed in a downstream fixed bed tubular reactor (Din = 13 mm) at atmospheric pressure (1 atm). To that end, 1.0 g (dp=0.3-0.5 mm) of the catalyst was used. Before analysis, the sample was reduced in a stream of 10 % H₂/Ar at 550 °C for 2 hours. During the storage step of the experiments that simulate feeds with CO₂ concentrations similar to those emitted in combustion processes (4-15 %), CO₂ was fed at 10 %/Ar for 1 min, followed by an Ar purge for 2 min to remove weakly adsorbed CO₂. Then, during the hydrogenation step, 10 % H₂/Ar was fed for 2 min, followed by an Ar purge for 1 min before starting the storage step again. Throughout the experiment, the total flow rate was 1200 ml min⁻¹, which corresponds to a GHSV of 110,000 h⁻¹. The experiments were carried out in the temperature range between 280 and 520 °C, every 40 °C. The composition of the exhaust gases was analysed continuously using the MultiGas 2030 FT-IR analyser for the quantitative analysis of CO₂, CH₄, CO and H₂O.

**Figure 5** shows the operation at 400 °C for the catalyst of example 1 (Ni-La₂O₃/CeO₂). In these experiments, during the adsorption cycle there is a stream of 1.4 % CO₂/Ar for 1 min. **Figure 5** also includes the CO₂ concentration profile in the feed (CO₂ bypass). As can be observed, CO₂ concentration at the reactor outlet is practically negligible at the beginning of the storage step. CO₂ is not detected until after 30 seconds, which assigns it to the progressive saturation of the CO₂ storage centres. In turn, H₂O is detected at the reactor outlet, which is desorbed due to the progressive storage of CO₂. Lastly, the hydrogenation period begins with the feed of a gas stream composed of 10 % H₂/Ar. The addition of H₂ in the gas stream reduces the stability of carbonates and favours their decomposition and subsequent conversion to CH₄ in the Ni⁰ sites. The formation of CH₄ is detected immediately once H₂ is admitted to the reactor, while the formation of H₂O is delayed around 20 seconds. This fact suggests that water is being adsorbed at the storage sites. Furthermore, a small, almost negligible production of CO (4 µmol) is observed during the step of CO₂ hydrogenation. Therefore, the results obtained confirm that the catalyst synthesised in example 1 reached an adequate CO₂ storage capacity (defined as the difference between what was fed into the bypass and what was observed at the outlet during the experiment), high stored CO₂ hydrogenation efficiency and high methane production, with a selectivity towards methane greater than 90 %. These results confirm the applicability of the materials claimed in the present invention as DFMs for efficient CO₂ methanation from streams of a highly varied nature. In fact, these materials have shown methane productions of up to 112 µmol · g⁻¹.

## Claims

1. A catalyst for the methanation of carbon monoxide, carbon dioxide or mixtures thereof, comprising:
- a support that comprises at least one metal oxide;
- at least one oxide of an element A, wherein the element A is a lanthanide element, an alkaline element, an alkaline earth element or mixtures thereof; and
- nanoparticles of an element B, wherein the element B is a noble metal, a transition metal or mixtures thereof, wherein said nanoparticles have a size between 1 nm and 8 nm and are distributed on the catalyst surface.

2. The catalyst according to claim 1, wherein the at least one metal oxide of the support is selected from oxides of group III elements, group IV elements, rare earth elements, lanthanides and mixed oxides thereof.

3. The catalyst according to any of claims 1 and 2, wherein the at least one oxide of the element A is selected from La₂O₃, BaO, CaO, SrO, Na₂O, K₂O, CeO and mixtures thereof.

4. The catalyst according to any of claims 1 to 3, wherein the element B of the nanoparticles is selected from Cu, Co, Mn, Fe, Ni, Ru, Rh, Pt, Pd, Au, Ag, Al, Mo and mixtures thereof.

5. The catalyst according to any of claims 1 to 4, wherein the nanoparticles of the element B have a size ≤5 nm.

6. A catalyst preparation method according to any of claims 1 to 5, comprising the steps of:
a) preparing an aqueous solution of metal precursors of an ABO₃ perovskite and citric acid, wherein the molar ratio of citrate to metal precursors is between 0.7 and 1.5, and wherein the pH of the aqueous solution is adjusted to a value between 1 and 9;
b) distributing the aqueous solution resulting from step (a) on a support by wet impregnation to obtain a supported perovskite precursor gel;
c) calcining the gel resulting from step (b) to obtain the supported perovskite; and
d) reducing the supported perovskite resulting from step (c) to give rise to the final catalyst.

7. The catalyst preparation method according to claim 6, wherein the molar ratio of citrate to metal cations of the metal precursors in step (a) is 1.1.

8. The catalyst preparation method according to any of claims 6 and 7, wherein the pH of step (a) is adjusted to a value between 5 and 9.

9. The catalyst preparation method according to any of claims 6 to 8, wherein the supported perovskite precursor gel resulting from step (b) is dried at a temperature between 80 °C and 150 °C.

10. The catalyst preparation method according to any of claims 6 to 9, wherein the supported perovskite precursor gel from step (c) is calcined at a temperature between 600 °C and 1200 °C.

11. The catalyst preparation method according to any of claims 6 to 10, wherein the supported perovskite from step (d) is reduced to a temperature between 500 °C and 900 °C.

12. A catalyst for the methanation of carbon monoxide, carbon dioxide or mixtures thereof, which can be obtained by the method according to any of claims 6 to 11, comprising:
- a support that comprises at least one metal oxide;
- at least one oxide of an element that forms the A cation of a starting ABO₃ perovskite, wherein said element is a lanthanide element, an alkaline element, an alkaline earth element or mixtures thereof; and
- nanoparticles of an element that forms the B cation of a starting ABO₃ perovskite, wherein said element is a noble metal, a transition metal or mixtures thereof, wherein said nanoparticles have a size between 1 nm and 8 nm and are distributed on the catalyst surface.

13. A method for the methanation of carbon monoxide, carbon dioxide or mixtures thereof, comprising bringing the catalyst into contact according to any of claims 1 to 5 and 12, with a flow stream of carbon monoxide, carbon dioxide or mixtures thereof and hydrogen at a temperature between 200 and 600 °C.

14. A dual function material for the adsorption and methanation of carbon monoxide, carbon dioxide or mixtures thereof, from diluted or concentrated streams, comprising the catalyst according to any of claims 1 to 5 and 12.

15. Use of the catalyst according to any of claims 1 to 5 and 12, in the adsorption and/or methanation of carbon monoxide, carbon dioxide or mixtures thereof.
